# FASCICULE DE BREVET EUROPEEN

(11) **EP 4 194 000 B1**
(45) Date de publication et mention de la délivrance du brevet: **23.07.2025**
(21) Numéro de dépôt: 22212008.1
(22) Date de dépôt: 07.12.2022
(51) Int. Cl.: A61K 36/185, A61P 17/04, A61P 17/06, A61P 17/08, A61P 37/06

(54) **EXTRAIT DE CISTUS MONSPELIENSIS ET COMPOSITIONS LE COMPRENANT POUR APAISER LA PEAU**
EXTRAKT AUS CISTUS MONSPELIENSIS UND ZUSAMMENSETZUNGEN DAMIT ZUR HAUTDEPAMERISIERUNG
CISTUS MONSPELIENSIS EXTRACT AND COMPOSITIONS COMPRISING SAME FOR SKIN SOOTHING

(30) Priorité: 07.12.2021 FR 2113075
(43) Date de publication de la demande: 14.06.2023
(73) Titulaire: Pierre Fabre Dermo-Cosmétique, 81500 Lavaur (FR)
(72) Inventeur: LESTIENNE, Fabrice, 81106 CASTRES (FR); LETI, Mathieu, 81106 CASTRES (FR)
(74) Mandataire: Regimbeau

(56) Documents cités:
- WO-A1-2008/053246
- WO-A1-2020/128223
- FR-A1- 3 074 421
- FR-A1- 3 095 588
- SAYAH K ET AL: "In vivoanti-inflammatory and analgesic activities ofCistus salviifolius(L.) andCistus monspeliensis(L.) aqueous extracts", SOUTH AFRICAN JOURNAL OF BOTANY - SUID-AFRIKAANS TYDSKRIFT VIRPLANTKUNDE, vol. 113, 2017, pages 160 - 163, XP085293250, ISSN: 0254-6299, DOI: 10.1016/J.SAJB.2017.08.015
- CHOI JAE EUN ET AL: "Skin neurogenic inflammation", SEMINARS IN IMMUNOPATHOLOGY, SPRINGER BERLIN HEIDELBERG, BERLIN/HEIDELBERG, vol. 40, no. 3, 30 April 2018 (2018-04-30), pages 249 - 259, XP036508477, ISSN: 1863-2297, [retrieved on 20180430], DOI: 10.1007/S00281-018-0675-Z

## Description

### DOMAINE TECHNIQUE DE L'INVENTION

La présente invention concerne un extrait de *Cistus monspeliensis* qui peut être utile dans la protection et/ou le traitement des peaux sensibles, de la dermatite atopique, notamment par une action combinée sur l'inflammation neurogène cutanée et le prurit. L'invention a également pour objets des compositions cosmétiques ou dermatologiques comprenant un tel extrait comme agent actif.

### ETAT DE LA TECHNIQUE

*Cistus monspeliensis* L. est un arbuste du pourtour méditerranéen communément appelé « Ciste de Montpellier » ou « Montpellier Rockrose ». Il colonise en France les terrains secs, les maquis et les garrigues des côtes méditerranéennes et de Corse. La plante, glutineuse, sécrète un exsudat collant et aromatique. Ses rameaux sont dressés et portent des feuilles persistantes, rugueuses, sessiles ou subsessiles, linéaires-lancéolées ou étroitement elliptiques, trinervées, à marges révolutées. Les inflorescences sont en cymes scorpioïdes unilatérales composées de fleurs de 2 à 3 cm de diamètre, présentant 5 pétales blancs, jamais maculés de pourpre. Les fruits sont des capsules arrondies, facilement écrasées par pression des doigts (Source : TISON J.-M. 2014, Flora Gallica - Flore de France. Biotope Editions, 1195).

Le Ciste de Montpellier fait partie des plantes dites « pionnières ». Sa capacité d'adaptation et sa résistance aux sols pauvres, au sel, ainsi qu'à la sécheresse, lui permettent de coloniser les terrains dégradés des zones côtières méditerranéennes, et de freiner leur érosion. Il s'installe souvent dans les anciennes terres cultivées ou les pâturages à l'abandon.

Plusieurs usages traditionnels de *Cistus monspeliensis* L. sont référencés dans le pourtour méditerranéen, notamment par voie topique. Au Maroc, les feuilles sont utilisées sous forme de cataplasmes appliqués sur les blessures, comme anti-diarrhéiques et pour ses propriétés anti-inflammatoires. L'usage du Ciste de Montpellier comme infusion, comme épice ou en cas d'asthme est reporté (Demetzos et al., 2001, Planta Medica 67 : 614-618). On trouve également des extraits de *Cistus monspeliensis* L. dans plusieurs références cosmétiques anti-âges.

Les parties aériennes de *Cistus monspeliensis* L. contiennent des diterpènes de type labdane parmi lesquels on peut citer l'acide 8-hydroxylabdan-15-oique ou l'oxyde de manoyle.

Des diterpènes de type clerodane sont également documentés comme par exemple l'acide 15-acetoxy-cis-clerodan-3-ene-18-oique (Papaefthimiou et al., 2014, Frontiers in Chemistry 2(35) : 1-19).

*Cistus monspeliensis* L. renferme également plusieurs familles de polyphénols parmi lesquels des flavanoïdes, des tanins, en particulier des tanins ellagiques, et des acides phénoliques (Santagati et al., 2008, Journal of Chromatographic Science 46(2) : 150-156). Les parties aériennes de *Cistus monspeliensis* L. sont par ailleurs source d'huile essentielle dont les constituants majeurs sont de type terpénique et phénolique (Loizzo et al, 2013, Food and Chemical Toxicology 59 :586-594).

Différents extraits de *Cistus monspeliensis* L. ont fait l'objet d'études pharmacologiques *in vitro* et *in vivo.* Les propriétés antimicrobiennes et antifongiques sont documentées (Bouamama et al., 2006, Journal of Ethnopharmacology 104 : 104-107) comme l'activité anti-Leishmania portée par les diterpènes du Ciste de Montpellier (Fokialakis et al., 2006, Biological and Pharmaceutical Bulletin 29(8) : 1775-1778).

L'activité antioxydante d'un extrait méthanolique de *Cistus monspeliensis* L. a été mise en évidence *in tubo* avec notamment une CI₅₀ de 3 µg/ml sur un test DPPH (2,2-diphényl 1-picrylhydrazyle). L'activité antioxydante d'un extrait aqueux, son activité protectrice vis-à-vis du clivage de l'ADN, et son activité inhibitrice de la peroxydation lipidique ont également été montrées. Par ailleurs, l'activité antioxydante de l'huile essentielle de *Cistus monspeliensis* L. a également été documentée dans le cadre d'une étude visant à évaluer son intérêt dans la prévention de maladies neurodégénératives (Loizzo et al., 2013).

Il a également été mis en évidence *in vitro* une action inhibitrice des enzymes alpha-glucosidase et alpha-amylase d'extraits aqueux et hydro-méthanolique de *Cistus monspeliensis* L., liant la plante à une potentielle activité hypoglycémiante (Sayah et al., 2017, BioMed Research International, 2789482 : 1-7).

Il a été rapporté, que des extraits hexaniques et aqueux de *Cistus monspeliensis* L. sont dotés d'une activité antiproliférative sur différentes lignées cellulaires (Angelopoulou et al., 2001, Planta Medica, 67(2) : 168-202), une activité myorelaxante *ex vivo* a été mise en évidence avec un extrait aqueux de *Cistus monspeliensis* L. (Attaguile et al., 2004, Journal of Ethnopharmacology, 92 : 245-250).

Une activité antiinflammatoire d'extraits aqueux et éthanolique de feuilles de *Cistus monspeliensis* L. a été révélée sur différents marqueurs précoces et génériques de l'inflammation sur des tests *in vitro* sur des cellules humaines (Taila et al., 2008, Journal of Pharmacie and Pharmacology, Suppl 1 : A-62, 158).

Les propriétés antiinflammatoires et analgésiques d'un extrait aqueux de partie aérienne de *Cistus monspeliensis* L. ont été mises en évidence *in vivo* par administration par voie orale à des souris et des rats (Sayah et al., 2017, South African Journal of Botany, 113 :160-163). Il est intéressant de noter que l'activité analgésique est obtenue après administration orale sur des modèles de douleur animaux conventionnels comme le test de contorsion par injection d'acide acétique.

Des extraits de *Cistus monspeliensis* L. ont fait l'objet de dépôts de brevets pour une utilisation cosmétique, notamment dans le domaine de l'antiâge. Le brevet FR2819718 cite l'extrait de *Cistus monspeliensis* parmi des extraits lipidiques destinés à un usage cosmétique pour lutter le contre le vieillissement de la peau. Le brevet FR2868307 divulgue un extrait de *Cistus monspeliensis* dans le traitement des rides associées aux contractions musculaires. Le brevet JP2011162504 cite qu'un extrait de *Cistus monspeliensis* induit une production d'ATP. La demande de brevet GB2443388 décrit une association d'algine hydrolysée avec au moins un extrait de *Lavandula stoechas, Helichrysum italicum* et *Cistus monspeliensis* obtenu par hydrodistillation sous vide assistée par micro-ondes dans le traitement de l'acné par voie topique.

Enfin, la demande de brevet WO 2020128223 concerne de nouvelles utilisations cosmétiques et dermatologiques d'un extrait de *Cistus monspeliensis* dans le maintien ou le renforcement de la fonction barrière via notamment le maintien ou l'augmentation de la différenciation cellulaire. Les effets divulgués dans cette demande de brevet ne concernent que la peau saine, les peaux dites sensibles, pathologiques telles que les peaux atopiques ou atteintes de dermatites étant explicitement exclues.

Ainsi à la connaissance des inventeurs, aucun document de l'art antérieur ne divulgue qu'un extrait de *Cistus monspeliensis L.* à fortiori pas un extrait des parties aériennes de cette plante, présente une activité dans la protection et/ou le traitement des peaux sensibles, de la dermatite atopique, notamment par une action combinée sur l'inflammation neurogène cutanée et le prurit.

La peau constitue une barrière contre les agressions extérieures, notamment chimiques, mécaniques ou infectieuses.

La peau est constituée de trois parties principales, l'une superficielle, l'épiderme, la partie interne, le derme et une couche plus profonde, l'hypoderme, qui interagissent.

L'épiderme humain naturel est composé principalement de trois types de cellules qui sont les kératinocytes, très majoritaires, les mélanocytes et les cellules de Langherans. Chacun de ces types cellulaires contribue par ses fonctions propres au rôle essentiel joué dans l'organisme par la peau, notamment le rôle de protection de l'organisme des agressions extérieures.

Le derme fournit à l'épiderme un support solide. C'est également son élément nourricier. Il est principalement constitué de fibroblastes au sein d'une matrice extracellulaire composée majoritairement d'une substance, dite substance fondamentale, contenant également le collagène et l'élastine. Ces composants sont synthétisés par les fibroblastes. On y trouve aussi des leucocytes, des mastocytes ou encore des macrophages tissulaires. Enfin, le derme est traversé par des vaisseaux sanguins et des fibres nerveuses, notamment des fibres sensorielles libres ou reliées à des capteurs.

Enfin l'hypoderme est la couche la plus profonde et la plus épaisse de la peau. Il s'inscrit dans la continuité du derme sans réelle séparation entre les deux tissus. L'hypoderme constitue un coussin amortisseur qui fait office de protection mécanique pour les structures sous-jacentes. Cette couche graisseuse permet également d'isoler le corps des variations thermiques. Si le derme peut être considéré comme une réserve d'eau, les graisses stockées au sein des adipocytes de l'hypoderme constituent une réserve d'énergie.

Un dysfonctionnement de l'organisation structurelle cellulaire épidermique, ou un défaut de la fonction barrière chimique de l'épiderme, peut se traduire par un état inflammatoire cutané.

Les dermatoses inflammatoires sont des affections de la peau et des muqueuses, souvent douloureuses, qui se caractérisent par des manifestations inesthétiques comme des rougeurs et des plaques de desquamation. Plusieurs pathologies sont regroupées sous la dénomination de dermatoses inflammatoires. On peut citer à titre d'exemples non limitatifs : la dermatite atopique, l'eczéma, le psoriasis, la rosacée, le lichen plan, les prurigos, les dermites séborrhéiques ou encore l'acné.

Un prurit peut être observé dans de nombreuses dermatoses cutanées, on parle de dermatoses prurigineuses comme la dermatite atopique, les dermatoses de contact, le psoriasis, le lichen plan, et des ectoparasitoses et piqûre d'insecte.

La dermatite atopique est la manifestation cutanée de l'atopie. C'est une dermatose inflammatoire chronique, survenant sur un terrain génétiquement déterminé. Elle touche 15 à 30% des enfants et 2 à 10% des adultes. Sa prévalence est en augmentation constante dans les pays industrialisés, elle a doublé voire triplé, au cours des trois dernières décennies et elle est maintenant considérée comme une préoccupation majeure de santé publique.

La dermatite atopique est souvent associée à d'autres désordres atopiques, tels que la rhinite allergique et l'asthme. Cette affection apparaît le plus souvent au cours de la petite enfance et se caractérise par des éruptions répétées pendant plusieurs années. Elle évolue par poussées entrecoupées de rémissions spontanées. Les lésions se caractérisent par une sécheresse cutanée importante associée à des manifestations inflammatoires : éruptions érythémateuses papuleuses, vésiculeuses, squameuses et très prurigineuses. Sur le plan histologique, la dermatite atopique se caractérise, comme bien des dermatoses, par une infiltration de lymphocytes, monocytes et de polynucléaires éosinophiles autour de petits vaisseaux et des capillaires ; sur le plan biochimique, elle se caractérise par l'expression de cytokines telles que la Thymic Stromal Lymphopoietin (TSLP), protéine majeure dans le déclenchement de l'inflammation associée à la dermatite atopique. Par ailleurs, il a été montré que les chimiokines, notamment l'interleukine 8 (IL8) et les médiateurs lipidiques de l'inflammation tels que la prostaglandine 6KF1α, sont fortement impliqués dans les dermatoses telles que la dermatite atopique et dans les maladies inflammatoires chroniques en général.

L'acné est une pathologie cutanée commune, résultat d'une inflammation des follicules pilo-sébacés due en grande partie à la colonisation de Cutibacterium acnes dans l'infundibulum (Dréno et al., JEADV 2018, 32 (Suppl 2) 5-14). Par ailleurs, il est également connu que la voie Th17 est fortement activée dans les lésions de patients présentant de l'acné (Kelhala et al., PLOS One, 9(8) : e105238, 2014).

Le prurit est défini comme une sensation déplaisante qui provoque le besoin de se gratter. Il émerge depuis peu la notion de pruricepteurs, récepteurs spécifiques permettant de percevoir le prurit, mais leur distinction de la famille des récepteurs nociceptifs reste encore débattue. En effet, ces pruricepteurs utilisent les fibres intra-épidermiques de type A δ et surtout celles de type C. Ils sécrètent des neuropeptides : la substance P, le « calcitonin gene related peptide » (CGRP) et le « vasoactive intestinal peptide » (VIP). Depuis peu, le rôle des protéases dans l'induction du prurit a été clairement établi. En effet, leur récepteur PAR-2 a été défini comme une grande voie d'activation du prurit. Il a également été identifié comme un acteur important dans de nombreuses pathologies cutanées inflammatoires ou dermatoses inflammatoires et en particulier la dermatite atopique.

Il a récemment été démontré l'implication de nouveaux récepteurs cellulaires du prurit non-histaminergique, les « Mas-related G protein-coupled receptor » (MRGPR), qui ont clairement été identifiés comme étant impliqués dans le prurit et l'inflammation.

L'inflammation neurogène cutanée se définit comme l'induction et/ou l'amplification d'un processus inflammatoire primaire par les terminaisons nerveuses, ainsi il s'agit d'une inflammation de la peau induite par l'activation des fibres nerveuses intra-épidermiques qui sécrètent des neuropeptides tels que la substance P. L'inflammation neurogène cutanée est particulièrement impliquée dans les peaux sensibles, les peaux intolérantes réactives voire dans des dermatoses inflammatoires prurigineuses. Depuis peu, le rôle des protéases (trypsine, cathepsine G, thrombine etc.) dans l'induction du prurit a été clairement établi. En effet, leur récepteur PAR-2 a été défini comme la deuxième grande voie d'activation du prurit. Les fibres nerveuses intradermiques interagissent directement ou indirectement avec les cellules cutanées et les cellules des systèmes endocrinien, lymphatique et immunitaire. Ces communications ont conduit à la définition d'un système neuro-immuno-endocrino-cutané. Pour fonctionner, ce système nécessite un langage commun constitué de molécules de différentes natures, les neuromédiateurs, les cytokines et des facteurs de croissance.

Ces molécules sont synthétisées et libérées par les cellules de la peau, les cellules immunitaires résidentes et recrutées, ainsi que par les terminaisons nerveuses intra-épidermiques. Les cellules épidermiques et dermiques peuvent également produire des neuromédiateurs, des enzymes, des neurotrophines, des cytokines, des chémokines et des facteurs de croissance. Ces médiateurs régulent l'innervation cutanée et la réponse inflammatoire. En cas d'inflammation, les cellules immunitaires en transit ou présentes constitutivement dans la peau, peuvent s'activer sous l'action de ces médiateurs sécrétées par les terminaisons nerveuses sensorielles et les cellules de la peau. Ce processus conduit à une seconde vague de libération de cytokines et neuromédiateurs, qui engendrent une boucle d'amplification de l'inflammation. Un nouveau concept émerge depuis peu, suggérant que les kératinocytes sont également des acteurs majeurs de l'inflammation neurogène cutanée. L'inflammation neurogène cutanée générée par des neuropeptides est impliquée dans la réactivité des peaux sensibles et aussi des peaux intolérantes. La notion de peau sensible reflète le niveau de sensibilité de la peau de chacun. S'il est possible d'avoir une peau sensible à tout âge, cela est extrêmement répandu chez les bébés et les personnes âgées. La peau des bébés a une épaisseur représentant environ un cinquième de celle de la peau des adultes, elle est par conséquent extrêmement sensible aux agressions chimiques, physiques et microbiennes, ainsi qu'aux rayons UV. La fonction de barrière de la peau des adultes, quant à elle, s'affaiblit progressivement avec l'âge, parallèlement au ralentissement des processus métaboliques. Le vieillissement de la peau entraîne peu à peu une déficience en lipides, ce qui la rend plus facilement irritable par les substances alcalines telles que le savon. Lorsque la peau présente un seuil de sensibilité très bas, c'est-à-dire qu'elle réagit de manière exacerbée à la moindre agression extérieure, on parlera de peau intolérante, voire de peau intolérante réactive. Les peaux intolérantes sont plus vulnérables aux agressions extérieures et se caractérisent par un inconfort quotidien et une forte irritabilité. Certains signes, plus ou moins marqués, permettent de les reconnaitre. La peau intolérante du visage présente par exemple des rougeurs et des picotements. Elle tire, chauffe ou démange. Elle peut également procurer des sensations de brûlure. Les peux intolérantes présentent généralement un terrain allergique et sont par conséquent, particulièrement sensibles aux composants des soins cosmétiques. La peau sensible est en fait une peau sujette aux picotements, échauffements, fourmillements et démangeaisons, parfois accompagnées de rougeurs. Ces sensations d'inconfort apparaissent de façon exacerbée en réaction à des stimuli qui ne déclencheraient pas d'irritation sur une peau normale.

Cette hyper-sensibilité de la peau résulte d'une diminution de son seuil de tolérance. Plus la peau est sensible, plus son seuil de tolérance est faible et lorsque le seuil de tolérance est au plus bas, on parlera de peau intolérante. Cette hyper-sensibilité peut s'expliquer par différents facteurs : - Une réaction inflammatoire qui se développe au contact de substances chimiques irritantes comme certains savons, les détergents ménagers ou la pollution ; le seuil déclenchant des ces substances permettant ainsi d'évoquer la peau sensible ou la peau intolérante. - Une altération de la fonction barrière de l'épiderme. Ce phénomène favorise alors une déshydratation de la peau et surtout la pénétration d'agents potentiellement irritants ; - Des facteurs psychologiques, comme le stress ; - Des facteurs hormonaux ; - Des facteurs physiques comme le soleil, les changements de température (chaud/froid), le vent, la climatisation, le chauffage, l'eau calcaire.

La présente invention a pour objet de répondre à ces besoins, c'est-à-dire de proposer des compositions qui protègent et/ou améliorent l'état des peaux sensibles, en diminuant l'inflammation neurogène cutanée d'une part et le prurit d'autre part.

### RESUME DE L'INVENTION

La présente invention porte sur un extrait de Cistus monspeliensis pour son utilisation dans la prévention et/ou le traitement des désordres cutanés liés à une inflammation neurogène cutanée, les désordres cutanés étant des dermatoses inflammatoires prurigineuses.

La présente invention porte également sur un extrait de *Cistus monspeliensis* pour son utilisation dans la prévention ou le traitement du prurit cutané.

Enfin la présente invention porte sur une composition dermatologique comprenant à titre de principe actif un extrait de *Cistus monspeliensis,* avec au moins un excipient dermatologiquement acceptable pour son utilisation dans la prévention et/ou le traitement des désordres cutanés liés à une inflammation neurogène cutanée, les désordres cutanés étant des dermatoses inflammatoires prurigineuses.

D'autres aspects de l'invention sont tels que décrits ci-dessous et dans les revendications.

### Définitions

Dans la présente invention, la plante *Cistus monspeliensis* L. pourra être désignée de manière abrégée par le terme *Cistus monspeliensis.*

Par « extrait de *Cistus monspeliensis »,* on entend désigner dans la présente invention, le produit d'extraction de tout ou partie de la plante *Cistus monspeliensis.*

Par « produit d'extraction », on entend au sens de la présente invention, le produit obtenu après extraction de la plante ou d'une partie de la plante, avec un solvant appelé solvant d'extraction, c'est-à-dire un produit présent dans le solvant d'extraction qui peut ensuite être éventuellement sous une forme concentrée ou sèche après évaporation partielle ou totale du solvant d'extraction. Il peut s'agir d'un extrait sec.

Par « extrait sec », on entend au sens de la présente invention, un extrait dépourvu de solvant d'extraction ou de support, ou en contenant uniquement à l'état de traces non significative. Un tel extrait sec contient ainsi uniquement de la matière issue de *Cistus monspeliensis.* Il peut contenir également des traces non significatives de solvant d'extraction.

Par « environ », on entend au sens de la présente invention, que la valeur concernée peut être inférieure ou supérieure de 10%, notamment de 5%, en particulier de 2%, plus particulièrement de 1% à la valeur indiquée.

Par « solvant moyennement polaire », on entend au sens de la présente invention, un solvant choisi dans le groupe constitué notamment des alcools en C1 à C5, des glycols comme le propylène glycol, le butylène glycol, le butanediol, ou le pentylène glycol, de glycérol, d'acétone, d'esters d'alkyles tels qu'acétate d'éthyle, acétate d'isopropyle de triéthyl citrate, de solvants miscibles à l'eau (un mélange hydro-alcoolique ou un mélange acétone/eau par exemple). Figurent également dans ce groupe des solvants alternatifs de type hydrotropes (molécules amphiphiles solubles dans l'eau et qui, à partir d'une concentration suffisante, peuvent extraire des composés moyennement polaires tels que décrits dans la caractérisation de l'extrait).

Par « solvant apolaire », il faut comprendre au sens de la présente invention, un solvant choisi par exemple parmi l'heptane, l'hexane, le limonène, l'oléate d'éthyle, les hydrocarbures halogénés (par exemple les hydrocarbures chlorés en C1 à C3 tels que le chloroforme ou le dichlorométhane), le CO₂ supercritique, un mélange CO₂ supercritique et éthanol et les mélanges de ces solvants. On peut citer également les solvants 100% biosourcés comme par exemple EcoXtract LIPOCOS (Fournisseur Pennakem Europe).

Par « extraction par CO₂ supercritique », on entend au sens de la présente invention, une extraction par du CO₂ dans un état supercritique, c'est-à-dire soumis à une pression et une température supérieures à son point critique. La température doit donc être supérieure à 31°C et la pression supérieure à 74 bar. Dans sa phase supercritique, le CO₂ est un solvant totalement neutre, non toxique, non polluant, non inflammable permettant l'extraction de composés lipophiles. Le CO₂ supercritique peut voir sa polarité modifiée par l'ajout d'un co-solvant polaire comme l'éthanol afin d'élargir la gamme de polarité des molécules extraites. L'extraction par CO₂ supercritique constitue ainsi une alternative privilégiée aux solvants pétrochimiques tels que l'hexane ou l'heptane.

Par « diterpène », on entend au sens de la présente invention des composés en C20 issus du métabolisme du 2^{E}, 6^{E}, 10^{E}-geranyldiphosphate. La structure des diterpènes est très variable, et dépendante de leur biogénèse.

Par « diterpène de type labdane », on entend au sens de la présente invention une classe de diterpènes présentant un squelette bicyclique auquel est rattaché une chaine à 6 atomes de carbone (cyclique ou acyclique) qui peut ou non contenir un atome d'oxygène.

Par « application topique », on entend au sens de la présente invention une application sur la peau (dont le cuir chevelu), et les muqueuses.

Par « cosmétiquement acceptable » ou « dermatologiquement acceptable », on entend au sens de la présente invention ce qui est utile dans la préparation d'une composition cosmétique ou dermatologique, qui est généralement sûr, non toxique et ni biologiquement ni autrement non souhaitable et qui est acceptable pour une utilisation cosmétique ou dermatologique.

Par « application topique », on entend au sens de la présente invention une application sur la peau (dont le cuir chevelu) et/ou les muqueuses.

### DESCRIPTION DETAILLEE DE L'INVENTION

De façon surprenante et inattendue, les inventeurs ont mis en évidence qu'un extrait, en particulier de parties aériennes, de *Cistus monspeliensis* L. possède une activité anti-prurit et modulatrice des voies de la nociception. Cet extrait est donc particulièrement intéressant dans la prévention et le traitement des des dermatoses inflammatoires prurigineuses, par exemple de la dermatite atopique ou du psoriasis.

De manière plus générale, l'extrait est tout particulièrement utile dans la protection et/ou le traitement des peaux sensibles.

L'extrait de *Cistus monspeliensis* utile dans le cadre de la présente invention peut être tel que défini ci-après.

### Extrait de Cistus monspeliensis

Dans le cadre de la présente invention, l'extrait de *Cistus monspeliensis* est obtenu à partir d'une ou plusieurs parties de la plante *Cistus monspeliensis* choisie(s) parmi la plante entière, les racines, les parties aériennes, les feuilles, les trichomes, les fruits, les fleurs, et/ou les tiges.

De façon avantageuse, l'extrait de *Cistus monspeliensis* est obtenu à partir de parties aériennes au stade végétatif, de parties aériennes fleuries ou de parties aériennes au stade de fructification de la plante *Cistus monspeliensis.*

De façon plus avantageuse, l'extrait de *Cistus monspeliensis* est obtenu à partir de parties aériennes fleuries de la plante *Cistus monspeliensis.*

La plante ou partie de plante *Cistus monspeliensis* peut être fraiche ou sèche, entière, coupée ou broyée puis soumise à une étape d'extraction. De façon avantageuse, la plante ou partie de plante *Cistus monspeliensis* est sèche et broyée avant d'être soumise à une étape d'extraction.

Dans un mode de réalisation de l'invention, la plante sèche broyée peut être mouillée dans de l'eau avant extraction.

Dans un mode de réalisation particulier de l'invention, l'extrait de *Cistus monspeliensis* est obtenu à partir d'une culture de cellules de *Cistus monspeliensis.*

Selon un mode de réalisation préféré de l'invention, l'extrait de *Cistus monspeliensis* est un extrait obtenu par extraction par CO₂ supercritique, en particulier un extrait obtenu par extraction par CO₂ supercritique avec ou sans ajout d'éthanol comme co-solvant, de parties aériennes fleuries de la plante *Cistus monspeliensis.*

Selon un mode de réalisation préféré de l'invention, l'extrait de *Cistus monspeliensis* selon l'invention, est susceptible d'être obtenu par un procédé tel que décrit ci-après.

Un procédé de préparation d'un extrait de *Cistus monspeliensis* utile dans le cadre de l'invention, comprend une étape d'extraction de tout ou partie de plante *Cistus monspeliensis* par un solvant moyennement polaire à apolaire, de préférence un solvant apolaire.

Dans un mode de réalisation, le solvant d'extraction pourra être choisi parmi le CO₂ supercritique avec ou sans co-solvant éthanol, l'hexane, l'heptane, des solvants agrosourcés de type EcoXtract LIPOCOS, l'oléate d'éthyle, l'acétate d'éthyle, l'acétate d'isopropyle, le triéthyl citrate, un glycol en C3 à C5, un alcool de C1 à C5, un mélange alcool/eau, une solution hydrotropique ou un mélange de ceux-ci.

Avantageusement, il s'agira de CO₂ supercritique avec ou sans co-solvant éthanol, ou d'acétate d'éthyle.

Avantageusement, il s'agira de CO₂ supercritique avec ou sans co-solvant éthanol.

De façon plus avantageuse, il s'agira de CO₂ supercritique avec co-solvant éthanol.

De façon encore plus avantageuse, ce mélange CO₂ supercritique/éthanol sera caractérisé par une proportion CO₂ supercritique/éthanol de 70 : 30 à 99 : 1 (m/m).

Et de façon encore plus avantageuse, le solvant apolaire est un mélange CO₂ supercritique/éthanol dans la proportion 92 : 8 (m/m).

Selon un autre mode de réalisation particulier, l'extraction par CO₂ supercritique est réalisée à une température comprise entre 32 et 55°C, à une pression comprise entre 200 et 600 bars. Le ratio poids de plante/volume de CO₂ supercritique pouvant varier de 1/10 à 1/100, pendant une durée de 1 minute à 48 heures. L'extraction peut être renouvelée de 2 à 3 fois.

Dans un autre mode de réalisation particulier, à la fin de l'extraction, l'extraction continue au CO₂ supercritique seul afin de sécher la plante.

Selon un autre mode de réalisation particulier, l'extrait obtenu est ensuite filtré afin de récupérer une phase liquide limpide exempte de particules. La phase liquide obtenue peut être plus ou moins concentrée, pouvant aller jusqu'à l'obtention d'un extrait sec.

Dans un autre mode de réalisation, un support peut être ajouté lors de l'étape de concentration de façon à obtenir un extrait contenant de 1 à 80% en poids d'extrait sec.

Le support peut être de la maltodextrine, du lactose, de la silice, de la glycérine, un glycol, une huile végétale, du triethyl citrate, de l'oléate d'éthyle, du dicaprylyl carbonate, de l'octyldodécanol, un hydrotrope, un mélange eau/solubilisant ou eau/tensioactif, ou tout autre support cosmétologiquement acceptable et solubilisant l'extrait, préférentiellement d'origine biosourcée comme par exemple des glycols biosourcés (1,2-pentanediol ; 1,3-butanediol ; 1,3-propanediol...), des acides gras estérifiés et également les hydrotropes comme par exemple les alkyls glycosides (Sepiclear, Apyclean, APXC4...).

Selon un mode de réalisation particulier, l'extrait de *Cistus monspeliensis* peut être décoloré, par exemple sur charbon actif.

### Compositions

L'extrait de *Cistus monspeliensis* peut être utilisé pour formuler des compositions cosmétiques ou dermatologiques. De telles compositions comprennent au moins un extrait de *Cistus monspeliensis* tel que décrit ci-avant, avec au moins un excipient cosmétiquement ou dermatologiquement acceptable. Les compositions cosmétiques ou dermatologiques, outre l'extrait de *Cistus monspeliensis* et un excipient cosmétiquement ou dermatologiquement acceptable, peuvent également contenir des agents tensioactifs, des agents complexants, des conservateurs, des antioxydants (comme les tocophérols), des agents stabilisants, des émulsifiants, des épaississants, des gélifiants, des humectants, des émollients, des oligo-éléments, des huiles essentielles, des parfums, des colorants, des agents matifiants, des filtres chimiques ou minéraux, des agents hydratants, des eaux thermales, etc.

Les compositions sont sous une forme adaptée à une utilisation par application topique. Les compositions cosmétiques ou dermatologiques pourront ainsi se présenter sous les formes qui sont habituellement connues pour une administration topique, c'est-à-dire notamment les lotions, les laits, les émulsions, les sérums, les baumes, les masques, les crèmes, les dispersions, les gels, les mousses, les sprays, les shampoings.

Selon un mode de réalisation particulier de l'invention, la composition dermo-cosmétique ou dermatologique, comprenant au moins un extrait de *Cistus monspeliensis* tel que décrit précédemment et au moins un excipient cosmétiquement ou dermatologiquement acceptable, comprend de 0,01 à 5% en poids, de préférence 0,01 à 4% en poids, de manière préférée de 0,02 à 2% en poids, de manière encore préférée de 0,02 à 1,5% d'extrait de *Cistus monspeliensis,* en poids d'extrait sec par rapport au poids total de la composition. Ce % en poids d'extrait sec s'entend hors poids de l'éventuel support de séchage, s'il y en a un, et ne concerne que l'extrait sec de plante.

### Utilisations de l'extrait de Cistus monspeliensis et des compositions comprenant un tel extrait

Ci-après, l'extrait de *Cistus monspeliensis* peut être tel que décrit en détail ci-dessus. Ci-après, le terme « composition » fait référence à une composition cosmétique ou dermatologique telle que décrite ci-dessus.

L'extrait de *Cistus monspeliensis* est tout particulièrement utile pour la protection et/ou le traitement des peaux sensibles. Ainsi, la présente invention concerne un extrait de *Cistus monspeliensis* ou une composition comprenant un tel extrait pour son utilisation dans la protection et/ou le traitement des peaux sensibles. L'extrait de *Cistus monspeliensis* ou les compositions comprenant un tel extrait apaise(nt) la peau et augmente(nt) le confort cutané. L'expression « peaux sensibles » désigne une peau présentant une réactivité accrue non immunologique à des stimulus physiques ou chimiques normalement bien tolérés. La réactivité accrue se manifeste typiquement par des signes d'inconfort cutané, tels que des picotements, des sensations de brûlure, des fourmillements, des tiraillements ou des démangeaisons.

L'extrait de *Cistus monspeliensis* réduit les sensations de picotements, les sensations de brûlures, les fourmillements, les tiraillements et/ou les démangeaisons cutanés. Ainsi, la présente invention porte également sur l'utilisation d'un extrait de *Cistus monspeliensis* ou d'une composition le comprenant pour réduire les sensations de picotements, les sensations de brûlures, les fourmillements, les tiraillements et/ou les démangeaisons. L'extrait de *Cistus monspeliensis* ou une composition le comprenant est tout particulièrement utile pour la prévention et/ou le traitement du prurit cutané. Ainsi, la présente invention porte sur un extrait de *Cistus monspeliensis* ou une composition comprenant un tel extrait pour son utilisation dans la prévention et/ou le traitement du prurit cutané.

L'invention concerne tout particulièrement un extrait de *Cistus monspeliensis* ou une composition comprenant un tel extrait pour son utilisation dans la prévention et/ou le traitement des dermatoses inflammatoires prurigineuses telles que la dermatite atopique, les dermatoses de contact, le psoriasis, le lichen plan, les ectoparasitoses et piqûres d'insecte.

L'invention concerne également une méthode dermatologique pour la prévention et/ou le traitement des désordres cutanés liés à/induits par une inflammation neurogène cutanée, en particulier des dermatoses inflammatoires prurigineuses, comprenant l'administration à une personne en ayant besoin d'une quantité efficace d'un extrait de *Cistus monspeliensis* ou d'une composition dermatologique comprenant au moins un extrait de *Cistus monspeliensis* avec au moins un excipient dermatologiquement acceptable.

L'invention concerne également une méthode cosmétique ou dermatologique pour la protection et/ou le traitement des peaux sensibles, en particulier en cas de dermatite atopique, notamment en réduisant l'inflammation neurogène cutanée et/ou en réduisant le prurit, comprenant l'administration à une personne en ayant besoin d'une quantité efficace d'un extrait de *Cistus monspeliensis* ou d'une composition cosmétique ou dermatologique comprenant au moins un extrait de *Cistus monspeliensis* avec au moins un excipient cosmétiquement ou dermatologiquement acceptable.

Les exemples qui suivent illustrent l'invention sans en limiter la portée.

### EXEMPLES

### Exemple 1 : extraction par CO₂ supercritique

300 grammes de parties aériennes fleuries sèches broyées de *Cistus monspeliensis* sont extraits par CO₂ supercritique à une pression de 250 bars et une température de 40°C à un débit de 10 kg/h pendant 180 minutes. Après détente jusqu'à pression atmosphérique et retour à température ambiante, l'installation est rincée à l'éthanol pour récupérer la totalité de l'extrait. L'extrait est filtré sur plaque de filtration K900 et l'éthanol est évaporé de manière à obtenir 34 grammes d'une cire verdâtre avec un rendement massique de 11%.

### Exemple 2 : extraction par CO₂ supercritique avec co-solvant éthanol

300 grammes de parties aériennes fleuries sèches broyées de *Cistus monspeliensis* sont extraits par CO₂ supercritique avec co-solvant éthanol 96° à une pression de 200 bars et une température de 40°C à un débit de 10 kg/h en CO₂ et 0,8 kg/h en éthanol 96° pendant 180 minutes. Après détente jusqu'à pression atmosphérique et retour à température ambiante, l'extrait est filtré sur plaque de filtration K900 et l'éthanol est évaporé de manière à obtenir 35 grammes d'une cire verdâtre avec un rendement massique de 12%.

### Exemple 3 : extraction par CO₂ supercritique avec co-solvant éthanol et décoloration au charbon actif

5 kilogrammes de parties aériennes fleuries sèches broyées de *Cistus monspeliensis* sont extraits par CO₂ supercritique avec co-solvant éthanol 96° à une pression de 200 bars et une température de 40°C à un débit de 10 kg/h en CO₂ et 0,8 kg/h en éthanol 96° pendant 180 minutes. Après détente jusqu'à pression atmosphérique et retour à température ambiante, l'extrait est filtré sur plaque de filtration K900 puis décoloré au charbon actif (0,2% p/v de solution). Après nouvelle filtration sur K900, l'éthanol est évaporé de manière à obtenir 524 grammes d'une cire orangée avec un rendement massique de 10%.

### Exemple 4 : extraction par CO₂ supercritique avec co-solvant éthanol et décoloration au charbon actif et mise sur support fluide

10 kilogrammes de parties aériennes fleuries sèches broyées de *Cistus monspeliensis* sont extraits par CO₂ supercritique avec co-solvant éthanol 96° à une pression de 200 bars et une température de 47°C à un débit de 40 kg/h en CO₂ et 3,2 kg/h en éthanol 96° pendant 7 heures. Après détente jusqu'à pression atmosphérique et retour à température ambiante, l'extrait est filtré sur plaque de filtration K900 puis décoloré au charbon actif (0,5% p/v de solution). Après nouvelle filtration sur K900, l'extrait est séché sur 1,2-pentanediol de manière à obtenir 5 kg d'extrait sous forme d'un liquide visqueux orangé.

### Exemple 5 : extraction à l'hexane

20 grammes de parties aériennes en fructification sèches broyées de *Cistus monspeliensis* sont extraits à température ambiante sous agitation par 200mL d'hexane pendant 1 heure dans un réacteur. L'extrait est ensuite filtré sur plaque de filtration K900 et le solvant est évaporé de manière à obtenir 2,2 grammes cire verdâtre avec un rendement massique de 11%.

### Exemple 6 : évaluation d'un extrait de Cistus monspeliensis selon l'invention dans un modèle de lymphocytes TH17

Il est clairement connu aujourd'hui que la réponse immunitaire adaptative joue un rôle clé dans l'apparition des lésions psoriasiques et les nouveaux traitements ciblant les interleukines IL-12, IL-23 ou IL-17 montrent une grande efficacité clinique chez les patients atteints de psoriasis en plaques modéré à sévère. Néanmoins, ces traitements systémiques restent contraignants et une approche topique offre toujours des avantages non négligeables : un traitement parfaitement limité épargnant la peau saine péri-lésionnelle, une meilleure hydratation cutanée avec protection contre le grattage, une meilleure acceptabilité des patients et une meilleure tolérance. En outre, les traitements locaux constituent la base de la prise en charge des psoriasis légers, c'est-à-dire les patients ayant une atteinte limitée à moins de 10% de la surface corporelle.

Le but de cette étude est d'évaluer l'activité potentielle d'un extrait de *Cistus monspeliensis* selon l'invention pour moduler la production de cytokines impliquées dans le psoriasis et la dermatite atopique. Pour se faire, des lymphocytes Th17 isolés et amplifiés à partir du sang circulant de donneurs sont utilisés comme modèle *in vitro* d'immunopathogénèse du psoriasis.

### Protocole :

L'extrait de *Cistus monspeliensis* testé est préparé selon l'exemple 3 et a été solubilisé dans du DMSO (diméthylsulfoxyde) puis mis dans le milieu de culture des Th17 purifiés, aux concentrations de 12,5 ; 25 et 50 µg/ml.

Les lymphocytes T sont triés à partir de cellules sanguines récupérées de poches de sang fournies par l'Etablissement Français du sang, pour obtenir le phénotype suivant : CD4+ CD45RA- CXCR3- CCR6+. Le nombre de lymphocytes Th17 ainsi obtenu est augmenté par une mise en culture de ces cellules en présence d'un mélange de cytokines (IL-2, anti-TNFα et anti-INFγ) pendant deux semaines.

Le test est réalisé sur des lymphocytes Th17 humains isolés et amplifiés en absence (conditions contrôle) ou en présence (traité) des composés à tester et sont stimulés ou non par les facteurs de stimulation anti-CD3/anti-CD28.

Les teneurs des cytokines pro-inflammatoires de Th17 (IL-17 et IL-22) sont quantifiées 24h après la stimulation.

Les lymphocytes sont traités avec les composés à tester 30 minutes avant la stimulation et pendant 24 heures.

La Nicardipine, bloqueur des canaux calciques non-sélectifs a été testée (10 µg/ml), solubilisée également dans le DMSO, est utilisée comme contrôle positif.

Chaque condition expérimentale a été réalisée en triplicata et répétée à partir de sang de donneurs différents.

Les cytokines pro-inflammatoires des lymphocytes Th17, IL-17 et IL-22 sont mesurées par des kits ELISA selon les instructions du fournisseur.

L'analyse statistique est déterminée sur le pourcentage d'inhibition après vérification de la normalité et de l'égalité des variances, en utilisant une ANOVA (ANnalysis Of Variance-analyse de variance) une voie suivie du test de Dunnett comme post test.

### Résultats

La libération d'IL-17 (pg/ml) après traitement des lymphocytes Th17 avec les billes anti-CD3/anti-CD28 est représentée dans le tableau 1. Il s'agit de billes dites « coatées », sur lesquelles sont fixées les anticorps cités, afin de stimuler les lymphocytes.

**Tableau 1 : effet d'un extrait de Cistus monspeliensis (extrait selon l'invention) sur la production d'IL-17**

| Groupes | Concentration | IL-17 (moyenne) (pg/ml) | Erreur Standard | % Inhibition | Stats VS. stim |
|---|---|---|---|---|---|
| Contrôle | - | 159 | 53 | - | P<0,001 |
| Stimulation | - | 1187 | 109 | - | - |
| Nicardipine | 10µg/ml | 721 | 63 | 43 | P<0,001 |
| Extrait selon l'invention | 12,5µg/ml | 537 | 187 | 58 | P<0,001 |
| | 25µg/ml | 671 | 42 | 47 | P<0,01 |
| | 50µg/ml | 371 | 151 | 79 | P<0,001 |

L'exposition des lymphocytes Th17 aux billes anti-CD3/anti-CD28 induit une augmentation marquée et statistiquement significative d'une libération d'IL-17 (tableau 1). La Nicardipine (10µg/ml) réduit de 43% cette production d'IL-17. Ce résultat fournit la preuve de la bonne réactivité du modèle des lymphocytes Th17 isolés et amplifiés, ce résultat permet de valider le test.

Le traitement des lymphocytes Th17 avec l'extrait de *Cistus monspeliensis* selon l'invention montre de façon surprenante une inhibition de la libération d'IL-17.

Cette inhibition apparait statistiquement significative aux 3 concentrations testées, avec près de 80% d'inhibition à 50µg/ml de *Cistus monspeliensis.*

La libération d'IL-22 (pg/ml) après traitement des lymphocytes Th17 avec les billes anti-CD3/anti-CD28 est représentée dans le tableau 2.

**Tableau 2 : effet d'un extrait de Cistus monspeliensis (extrait selon l'invention) sur la production d'IL-22**

| Groupes | Concentration | IL-22 (moyenne) (pg/ml) | Erreur Standard | % Inhibition | Stats VS. stim |
|---|---|---|---|---|---|
| Contrôle | - | 92 | 16 | - | P<0,05 |
| Stimulation | - | 945 | 357 | - | - |
| Nicardipine | 10µg/ml | 440 | 88 | 37 | NS |
| Extrait selon l'invention | 12,5µg/ml | 252 | 72 | 51 | NS |
| | 25µg/ml | 401 | 114 | 46 | NS |
| | 50µg/ml | 270 | 52 | 64 | P<0,01 |

L'exposition des lymphocytes Th17 aux billes anti-CD3/anti-CD28 induit une augmentation marquée et statistiquement significative d'une libération d'IL-22 (tableau 2). La Nicardipine (10µg/ml) réduit la production d'IL-22, mais l'inhibition n'atteint pas le seuil de significativité statistique. En effet dans le groupe stimulé, une grande variabilité des résultats est observée.

Le traitement des lymphocytes Th17 avec l'extrait de de *Cistus monspeliensis* selon l'invention montre également une inhibition de la libération d'IL-22. En effet, à la concentration de 50µg/ml l'extrait de *Cistus monspeliensis* selon l'invention réduit significativement de 64% la production d'IL-22. Aux concentrations inférieures, l'effet inhibiteur n'atteint pas la significativité statistique, bien que l'effet atteint 50%.

Les inventeurs ont ainsi montré que les lymphocytes Th17 stimulés induisaient une libération massive d'IL-17 et d'IL-22. Dans ce modèle, les inventeurs démontrent qu'un extrait de *Cistus monspeliensis* selon l'invention est très efficace en limitant cette libération d'interleukines inflammatoires, marqueur clé dans le psoriasis, l'acné et la dermatite atopique, démontrant un rôle protecteur dans ces pathologies, notamment par un effet apaisant.

### Exemple 7 : évaluation d'un extrait de Cistus monspeliensis sur la production d'IL-8 et de TSLP dans un modèle de dermatite atopique

Les inventeurs ont évalué les effets d'un extrait de *Cistus monspeliensis* sur la production de cytokines, induite par un mélange de ligands inflammatoires sur des kératinocytes épidermiques humains normaux. Ce mélange inflammatoire est utilisé pour mimer les deux phases de la dermatite atopique à savoir la phase d'initiation et la phase chronique. Dans ce modèle, la cytokine TSLP (Thymic Stromal LymphoPoietin) qui joue un rôle crucial dans le développement de la dermatite atopique (Ziegler and Artis, 2010, Nat. Immunol. 11 (4) : 289-293) est produite par ce mélange inflammatoire. Il-8 est également fortement induite par ces conditions. Le but de cette étude est d'évaluer le rôle putatif d'un extrait de *Cistus monspeliensis* dans l'inhibition de la sécrétion de TSLP et d'IL-8.

L'extrait de *Cistus monspeliensis* a été préparé selon l'exemple 3, il a été testé à 3, 10 et 30 µg/ml, solubilisé dans 0,03% DMSO et mis dans le milieu de culture des kératonocytes.

Les kératinocytes épidermiques humains normaux sont pré-incubés pendant 1 heure dans un milieu d'essai contenant ou pas (contrôle) le composé à tester ou le produit de référence (Bafilomycine à 30 nM). Ces cellules sont ensuite stimulées avec le mélange inflammatoire composé de Poly(I:C), PAM3CSK4, IL-4 et IL-13. Poly(I:C) est un ligand TLR (Toll-Like Receptors), PAM3CSK4 est un agoniste TLR2/TLR1, et IL-4 et IL-13 sont deux interleukines de type Th2.

Une condition contrôle, non-stimulée est également réalisée en parallèle.

Les cellules sont ensuite incubées pendant 24 heures.

Trois expériences indépendantes sont réalisées.

Le surnageant de culture est alors enlevé, centrifugé et congelé à -20°C. Les productions de TSLP et d'IL-8 sont quantifiés par ELISA, selon les instructions du fournisseur (R&D Systems).

La détection de fluorescence est réalisée en utilisant le système Flex station 3 (Molecular Devices). La quantification de fluorescence est faite grâce à l'algorithme Soft Max Pro qui fournit des Aires sous la Courbe pour chaque condition expérimentale.

Toutes les expériences sont faites en triplicate.

Une analyse statistique est réalisée pour évaluer la condition stimulée versus la condition non-traitée en utilisant un test T non-apparié. La comparaison inter-groupe est faite par une ANOVA à une voie en mesures répétées suivies par un post test de Dunnett. Ces analyses statistiques sont produites par le logiciel PRISM.

### Résultats

Les résultats sur la production de TSLP sont résumés dans le tableau 3.

Le traitement des kératinocytes épidermiques humains normaux avec le mélange inflammatoire induit une forte, statiquement significative et reproductible production de TSLP. Ces résultats étaient attendus et valident le test.

En présence de DMSO (0,03%), la production de TSLP n'est statistiquement pas affectée, ce qui montre que le DMSO n'est pas responsable de l'effet observé.

**Tableau 3 : effet d'un extrait de Cistus monspeliensis (extrait selon l'invention) sur la production de TSLP**

| | Concentration | Moyenne ± sem*(pg/ml) | Inhibition (%) | p |
|---|---|---|---|---|
| Contrôle | - | 4 ± 2 | - | - |
| Stimulation | - | 138 ± 19 | - | - |
| DMSO | 0,03% | 169 ± 21 | - | NS |
| Extrait selon l'invention | 3µg/ml | 121 ± 14 | 29 | NS |
| | 10µg/ml | 99 ± 9 | 42 | P<0,05 |
| | 30µg/ml | 41 ± 4 | 77 | P<0,01 |

| | | | | |
|---|---|---|---|---|
| * sem : erreur standard de la moyenne NS : Non significatif | | | | |

Lorsque les kératinocytes épidermiques humains normaux sont incubés en présence d'un extrait de *Cistus monspeliensis,* les inventeurs mettent en évidence une inhibition concentration-dépendante de la production de TSLP.

Cette inhibition apparait statistiquement significative à partir de 10µg/ml de *Cistus monspeliensis.* A 30µg/ml l'extrait de *Cistus monspeliensis* réduit de près de 80% la production de TSLP induite par le mélange inflammatoire.

Les résultats sur la production d'IL-8 sont résumés dans le tableau 4.

Le traitement des kératinocytes épidermiques humains normaux avec le mélange inflammatoire induit une forte, statiquement significative et reproductible production d'IL-8. Ces résultats étaient attendus et valident le test.

En présence de DMSO (0,03%), la production de TSLP n'est statistiquement pas affectée.

**Tableau 4 : effet d'un extrait de Cistus monspeliensis (extrait selon l'invention) sur la production d'IL-8**

| | Concentration | Moyenne ± sem* (pg/ml) | Inhibition (%) | p |
|---|---|---|---|---|
| Contrôle | - | 0 ± 0 | - | - |
| Stimulation | - | 36 780 ± 4 146 | - | - |
| DMSO | 0,03% | 39 842 ± 3 844 | - | NS |
| Extrait selon l'invention | 3µg/ml | 36 832 ± 4 679 | 9 | NS |
| | 10µg/ml | 15 616 ± 1 111 | 61 | P<0,01 |
| | 30µg/ml | 1 599 ± 283 | 96 | P<0,01 |

| | | | | |
|---|---|---|---|---|
| * sem : erreur standard de la moyenne NS : Non significatif | | | | |

Lorsque les kératinocytes épidermiques humains normaux sont incubés en présence d'un extrait de *Cistus monspeliensis,* les inventeurs mettent en évidence une inhibition concentration-dépendante et très marquée de la production d'IL-8.

Cette inhibition apparait statistiquement significative à partir de 10µg/ml de *Cistus monspeliensis.* A 30µg/ml l'extrait de *Cistus monspeliensis* réduit presque totalement (96% d'inhibition) la production de d'IL-8 induite par le mélange inflammatoire.

Les inventeurs mettent donc clairement en évidence qu'un extrait de *Cistus monspeliensis* est capable de réduire fortement les productions de TSLP et d'IL-8 induites par un mélange de ligands inflammatoires qui mime un environnement de dermatite atopique.

Ainsi les inventeurs démontrent qu'un extrait de *Cistus monspeliensis* est doté d'effets apaisant et soulageant dans la dermatite atopique.

### Exemple 8 : évaluation d'un extrait de Cistus monspeliensis sur des récepteurs impliqués dans la voie de signalisation du prurit

Les inventeurs ont évalué les effets d'un extrait de *Cistus monspeliensis* sur deux cibles étroitement reliées au prurit, PAR-2 et MRGPRX2.

Les PAR-2 sont des récepteurs couplés aux protéines G. PAR-2 module les réponses inflammatoires, joue un rôle dans l'obésité, le métabolisme et les cancers. PAR-2 agit comme un senseur pour des enzymes protéolytiques générées pendant l'infection. PAR-2 serait retrouvé dans les kératinocytes épidermiques au niveau de la couche *stratum granulosum.* Des PAR-2 fonctionnels sont également exprimés dans plusieurs types de cellules immunitaires, telles que les éosinophiles, les neutrophiles, les monocytes, les macrophages, les cellules dendritiques, les mastocytes et les cellules T. Le PAR-2 est activé par clivage protéolytique par la tryptase qui est la protéase principale des cellules mastocytaires et la kallikréine qui est impliquée dans des processus inflammatoires et d'homéostasie épidermique. De plus, le clivage protéolytique de PAR-2 induit une signalisation calcique s'il est exprimé dans les neurones participant à la voie afférente. L'activation de MRGPRX2 (Mast-Related G-Protein coupled Receptor) entraine une dégranulation des cellules mastocytaires avec par la suite des réactions pseudo-allergiques. Afin de déterminer les éventuelles activités antagonistes ou agonistes sur ces cibles, ces récepteurs recombinants ont été exprimés dans un modèle hétérologue et couplés à une voie de second messager.

L'extrait de *Cistus monspeliensis* a été préparé selon l'exemple 3, il a été testé à dix concentrations allant de 3,0 ng/ml à 100 µg/ml solubilisé dans du DMSO.

L'agoniste de référence pour PAR-2 est le peptide SLIGRL-NH₂ avec une valeur de CE₅₀ à 524 nM.

L'agoniste de référence pour MRGPRX2 est le neuropeptide Cortistatine-17 avec une valeur de CE₅₀ à 269 nM.

Les activités agonistes et antagonistes sur les récepteurs humains PAR-2 et MRGPRX2 ont été testées sur des tests à l'Aequorine.

Afin de valider la spécificité des activités antagonistes observées, une première activité agoniste a été testée sur la lignée cellulaire parentale, CHO-K1 mt aequorin, pour tous les composés testés sur ces cibles.

Les cellules recombinantes ont été cultivées pendant 18 heures avant le test dans des milieux sans antibiotiques, puis détachées par un rinçage doux avec du PBS-EDTA (5 mM EDTA), récupérées par centrifugation et remises en suspension dans du "tampon de test". Les cellules ont été incubées à température ambiante pendant au moins 4h avec la luciférine : Coelenterazine h (Molecular Probes). Des courbes dose-réponse avec les composés de référence ont été réalisées avant de tester les composés.

Pour le test agoniste, 50µl de suspension cellulaire ont été injectés sur 50µl de composé à tester ou d'agoniste de référence dans une plaque 96 puits. Après une incubation de 15 min après la première injection, 100µl de l'agoniste de référence à une concentration correspondant à sa CE₈₀ ont été injectés sur les 100µl du mélange de suspension cellulaire et de composé à tester, pour le test des antagonistes.

Trois expériences (N=3) ont été réalisées à des jours différents en double exemplaire (n=2).

Les composés ont été testés pour leur activité agoniste et antagoniste au niveau des récepteurs humains PAR-2 et MRGPRX2 sur des tests à l'Aequorine.

L'émission de lumière résultante a été enregistrée à l'aide du Functional Drug Screening System 6000 de Hamamatsu (FDSS 6000).

L'activité agoniste des composés testés a été exprimée en pourcentage de l'activité de l'agoniste de référence à sa concentration CE₁₀₀. L'activité antagoniste du composé testé a été exprimée en pourcentage de l'inhibition de l'activité de l'agoniste de référence à sa concentration CE₈₀.

Chaque jour d'expérimentation et avant de tester les composés, les composés de référence ont été testés à plusieurs concentrations en double (n=2) pour obtenir une courbe dose-réponse et une estimation des valeurs CE₅₀ et/ou CI₅₀.

Les valeurs de référence ainsi obtenues pour le test ont été comparées aux valeurs historiques obtenues pour le même récepteur et utilisées pour valider la session expérimentale.

Une session n'était considérée comme valide que si la valeur de référence se trouvait dans un intervalle de 0,5 log par rapport à la valeur historique. Pour les déterminations répétées, la variabilité maximale tolérée dans le test était de ± 20% autour de la moyenne.

Les données de dose-réponse des composés testés ont été analysées avec le logiciel XLfit (IDBS) en utilisant la régression non linéaire appliquée à un modèle dose-réponse sigmoïde.

L'activité agoniste des composés testés est exprimée en pourcentage de l'activité de l'agoniste de référence à sa concentration CE₁₀₀. L'activité antagoniste du composé testé est exprimée en pourcentage de l'inhibition de l'activité de l'agoniste de référence à sa concentration CE₈₀.

Les moyennes et les ESM (écart standard à la moyenne) ont été calculées avec les données obtenues sur les trois expériences séparées.

### Résultats

### Activité PAR-2

Il a été vérifié que l'extrait de *cistus monspeliensis* ne présentait ni une activité sur la lignée parentale CHO-K1 ni une activité agoniste sur la lignée recombinante CHO-K1 mt aequorin.

Les résultats sur l'activité antagoniste de l'extrait de *cistus monspeliensis* selon l'invention sur PAR-2 sont présentés dans le tableau 5.

**Tableau 5 : activité antagoniste de l'extrait de cistus monspeliensis selon l'invention sur PAR-2**

| | % d'inhibition moyennée à la concentration maximale | CI₅₀ (µg/ml) |
|---|---|---|
| Valeur individuelle | 90,5 | 26,8 |
| | 96,7 | 21,2 |
| | 97,1 | 14,0 |
| Moyenne | 94,8 ± 2,1 | 20,7 ± 3,7 |

Les inventeurs démontrent ainsi clairement qu'un extrait de *Cistus monspeliensis* selon l'invention présente une activité antagoniste sur PAR-2 (CI₅₀ de 20 µg/ml).

### Activité MRGPRX2

Il a été vérifié que l'extrait de *cistus monspeliensis* ne présentait ni une activité sur la lignée parentale CHO-K1 ni une activité agoniste sur la lignée recombinante CHO-K1 mt aequorin.

Les résultats sur l'activité antagoniste de l'extrait de *cistus monspeliensis* selon l'invention sur MRGPRX2sont présentés dans le tableau 6.

**Tableau 6 : activité antagoniste de l'extrait de cistus monspeliensis selon l'invention sur MRGPRX2**

| | % d'inhibition moyennée à la concentration maximale | CI₅₀ (µg/ml) |
|---|---|---|
| Valeur individuelle | 96,7 | 20,8 |
| | 96,9 | 34,1 |
| | 98,2 | 13,7 |
| Moyenne | 97,3 ± 0,5 | 22,9 ± 6,0 |

Les inventeurs démontrent ainsi clairement qu'un extrait de *Cistus monspeliensis* selon l'invention présente une activité antagoniste sur MRGPRX2 (CI₅₀ de 23 µg/ml).

Dans cette étude, les inventeurs ont pu mettre en évidence qu'un extrait de *Cistus monspeliensis* selon l'invention était doté de propriétés antagonistes sur les récepteurs PAR-2 et MRGPRX2, révélant un effet apaisant, notamment dans un contexte pruritogène.

### Exemple 9 : évaluation d'un extrait de Cistus monspeliensis sur un modèle de neurones sensoriels

Le but de cette étude est d'évaluer les propriétés modulatrices d'un extrait de *Cistus monspeliensis* dans l'inflammation neurogène cutanée.

Pour se faire, les inventeurs ont développé une approche expérimentale *in vitro.* Un modèle original de neurones sensoriels humains a été développé après une reprogrammation cellulaire de cellules souches pluripotentes humaines. Ce modèle permet d'étudier les propriétés inhibitrices de la libération de la substance P par les composés à tester, étudié après la stimulation. Ce neuropeptide, la substance P, étant le principal neuromédiateur impliqué dans les voies de la sensibilisation dans l'inflammation neurogène, la douleur et le prurit.

L'étape initiale est la reprogrammation de cellules souches pluripotentes humaines par l'utilisation successive de différents cocktails de facteurs de croissance.

Les caractérisations phénotypiques et morphologiques ont permis de vérifier le pattern des neurones sensoriels avec notamment l'expression des récepteurs spécifiques tels que TrKA, 5HT-2, P2RX3 ou des canaux tels que TRPV1, Nav1.7, Nav1.8, TRPM8 avec une colocalisation avec des marqueurs neuronaux (Tuj 1, Brn3A). Ces caractérisations permettent d'établir une durée de reprogrammation pour atteindre un pattern optimal qui est entre J40 et J47 après le début des traitements avec le cocktail des facteurs de croissance.

### Conditions expérimentales et traitement

L'essai est réalisé sur le modèle de neurones sensoriels en culture dans un mélange de milieu (RPMI/N2/B27/Glutamax) en absence (conditions contrôle) ou en présence (groupes traités) des produits à tester. Ces deux conditions sont testées en présence de Vératridine ou en son absence. La Vératridine (testée à 3µM, diluée dans du DMSO, concentration molaire finale 0,006%) est un alcaloïde polycyclique extrait du rhizome de la liliacée Veratrum album. Elle entraine une activation persistante des canaux sodiques voltage-dépendants. Les niveaux du neuropeptide qu'est la substance P sont quantifiés dix minutes après l'ajout de Veratridine.

Les neurones sensoriels sont traités pendant trois heures avec un extrait de *Cistus monspeliensis* préparé selon l'exemple 3, et testé aux concentrations de 3 et 30µg/ml dans le mieux de culture des neurones, et pendant 10 minutes sous stimulation (par la Vératridine). Simultanément, dans un autre groupe, les neurones sensoriels sont traités par la lidocaïne (un bloqueur des canaux sodiques voltage-dépendants) à 100 µM (diluée dans l'éthanol, concentration molaire finale 0,1%), utilisée comme contrôle positif. Chaque condition expérimentale est réalisée sur deux lots de neurones sensoriels séparés, et répétée à des temps distincts entre J40 et J47 afin d'avoir des expériences à n=2.

La substance P libérée est mesurée dans les milieux d'incubation du groupe non traité (contrôle) et des groupes traités après ou non la stimulation de Vératridine. Elle est quantifiée par ELISA selon les instructions du fournisseur.

Le niveau de substance P (pg/ml) est calculé par interpolation à partir d'une courbe standard. Les données brutes et les pourcentages d'inhibition sont analysés grâce aux logiciels Excel et GraphPad Prism de Microsoft. La normalité des données a été évaluée par les tests de normalité Shapiro-Wilk et Kolmogorov-Smirnov. Pour valider les expériences, un test de Student apparié est réalisé entre les conditions non-stimulées et stimulées. Enfin pour valider l'activité du composé de référence et du produit testé, des comparaisons statistiques inter-groupes sont réalisées à partir des données brutes par une ANOVA à une voie associée à un test Dunnett comme post-test.

### Résultats

La Vératridine à 3µM, comme attendu, augmente fortement et significativement la libération de substance P. La lidocaïne, testée à 100µM, réduit significativement la libération de substance P induite par la stimulation par la Vératridine. Ces résultats étaient attendus, ils permettent de valider ce test.

Les résultats de cette libération de substance P pour l'ensemble des groupes sont résumés dans le tableau 7.

**Tableau 7 : étude de la libération de substance P**

| | | Données brutes | |
|---|---|---|---|
| Groupes | Concentration | Moyenne | ESM |
| Témoin (contrôle) | | 47,67 | 16,71 |
| Vératridine | | 96,26 | 19,66 |
| Lidocaïne | 100µM | 34,76 | 14,23 |
| Extrait de *Cistus monspeliensis* | 3µg/ml | 91,97 | 35,24 |
| | 30µg/ml | 44,83 | 8,31 |

L'extrait de *Cistus monspeliensis* testé à 3µg/ml n'a pas d'effet sur la libération de substance P, en revanche à 30 µg/ml, il réduit significativement cette libération (P<0,05).

Ces résultats démontrent l'intérêt d'un extrait de *Cistus monspeliensis* selon l'invention pour moduler les voies de la nociception.

Avec l'ensemble de ces résultats, les inventeurs démontrent qu'un extrait de *Cistus monspeliensis* selon l'invention est utile dans le traitement de la dermatite atopique grâce son action anti-prurit, et modulateur des voies de la nociception. Cet extrait de *Cistus monspeliensis* selon l'invention est donc particulièrement recommandé pour son activité apaisante sur les peaux sensibles.

## Revendications

1. Extrait de *Cistus monspeliensis* pour son utilisation dans la prévention et/ou le traitement des désordres cutanés liés à une inflammation neurogène cutanée, les désordres cutanés étant des dermatoses inflammatoires prurigineuses.

2. Extrait de *Cistus monspeliensis* pour son utilisation selon la revendication 1, dans laquelle la dermatose inflammatoire prurigineuse est la dermatite atopique.

3. Extrait de *Cistus monspeliensis* pour son utilisation selon la revendication 1 ou 2, **caractérisé en ce que** l'extrait de *Cistus monspeliensis* est obtenu à partir des parties aériennes fleuries de la plante.

4. Extrait de *Cistus monspeliensis* pour son utilisation selon l'une quelconque des revendications 1 à 3, **caractérisé en ce que** l'extrait de *Cistus monspeliensis* est obtenu par extraction par CO2 supercritique, de préférence avec ajout d'éthanol comme co-solvant.

5. Extrait de *Cistus monspeliensis* pour son utilisation selon la revendication 1, pour son utilisation dans la prévention ou le traitement du prurit cutané.

6. Composition dermatologique comprenant à titre de principe actif un extrait de *Cistus monspeliensis,* avec au moins un excipient dermatologiquement acceptable, pour son utilisation dans la prévention et/ou le traitement des désordres cutanés liés à une inflammation neurogène cutanée, les désordres cutanés étant des dermatoses inflammatoires prurigineuses.

7. Composition dermatologique pour son utilisation selon la revendication 6, dans laquelle la dermatose inflammatoire prurigineuse est la dermatite atopique.

8. Composition dermatologique pour son utilisation selon la revendication 6 ou 7, **caractérisé en ce que** l'extrait de *Cistus monspeliensis* est obtenu à partir des parties aériennes fleuries de la plante.

9. Composition dermatologique pour son utilisation selon l'une quelconque des revendications 6 à 8, **caractérisé en ce que** l'extrait de *Cistus monspeliensis* est obtenu par extraction par CO2 supercritique.

10. Composition dermatologique pour son utilisation selon l'une quelconque des revendications 6 à 9, **caractérisé en ce que** l'extrait de *Cistus monspeliensis* est obtenu par extraction par CO2 supercritique avec ajout d'éthanol comme co-solvant.

11. Composition dermatologique pour son utilisation selon l'une quelconque des revendications 6 à 10, **caractérisée en ce qu'**elle comprend 0,01 à 5%, de préférence 0,02 à 2% d'extrait de *Cistus monspeliensis* en poids d'extrait sec par rapport au poids total de la composition.

## Patentansprüche

1. Extrakt aus *Cistus monspeliensis* für seine Verwendung bei der Vorbeugung und/oder Behandlung von Hauterkrankungen im Zusammenhang mit einer neurogenen Hautentzündung, wobei die Hauterkrankungen juckende entzündliche Dermatosen sind.

2. Extrakt aus *Cistus monspeliensis* für seine Verwendung nach Anspruch 1, wobei die juckende entzündliche Dermatose die atopische Dermatitis ist.

3. Extrakt aus *Cistus monspeliensis* für seine Verwendung nach Anspruch 1 oder 2, **dadurch gekennzeichnet, dass** der Extrakt aus *Cistus monspeliensis* aus den blühenden oberirdischen Teilen der Pflanze gewonnen wird.

4. Extrakt aus *Cistus monspeliensis* für seine Verwendung nach einem der Ansprüche 1 bis 3, **dadurch gekennzeichnet, dass** der Extrakt aus *Cistus monspeliensis* durch Extraktion mit superkritischem CO₂, vorzugsweise unter Zugabe von Ethanol als Co-Lösungsmittel, gewonnen wird.

5. Extrakt aus *Cistus monspeliensis* für seine Verwendung nach Anspruch 1 für seine Verwendung bei der Vorbeugung oder Behandlung von Hautjucken.

6. Dermatologische Zusammensetzung, die als Wirkstoff einen Extrakt aus *Cistus monspeliensis* mit mindestens einem dermatologisch verträglichen Trägerstoff enthält, für ihre Verwendung bei der Vorbeugung und/oder Behandlung von Hauterkrankungen im Zusammenhang mit einer neurogenen Hautentzündung, wobei die Hauterkrankungen juckende entzündliche Dermatosen sind.

7. Dermatologische Zusammensetzung für ihre Verwendung nach Anspruch 6, wobei die juckende entzündliche Dermatose die atopische Dermatitis ist.

8. Dermatologische Zusammensetzung für ihre Verwendung nach Anspruch 6 oder 7, **dadurch gekennzeichnet, dass** der Extrakt aus *Cistus monspeliensis* aus den blühenden oberirdischen Teilen der Pflanze gewonnen wird.

9. Dermatologische Zusammensetzung für ihre Verwendung nach einem der Ansprüche 6 bis 8, **dadurch gekennzeichnet, dass** der Extrakt aus *Cistus monspeliensis* durch Extraktion mit superkritischem CO₂ gewonnen wird.

10. Dermatologische Zusammensetzung für ihre Verwendung nach einem der Ansprüche 6 bis 9, **dadurch gekennzeichnet, dass** der Extrakt aus *Cistus monspeliensis* durch Extraktion mit superkritischem CO₂ unter Zugabe von Ethanol als Co-Lösungsmittel gewonnen wird.

11. Dermatologische Zusammensetzung für ihre Verwendung nach einem der Ansprüche 6 bis 10, **dadurch gekennzeichnet, dass** sie 0,01 bis 5 Gew.-%, vorzugsweise 0,02 bis 2 Gew.-% Extrakt aus *Cistus monspeliensis* als Trockenextrakt, bezogen auf das Gesamtgewicht der Zusammensetzung, umfasst.

## Claims

1. *Cistus monspeliensis* extract for use in the prevention and/or treatment of skin disorders associated with neurogenic skin inflammation, the skin disorders being pruritic inflammatory dermatoses.

2. *Cistus monspeliensis* extract for use according to claim 1, wherein the pruritic inflammatory dermatosis atopic dermatitis.

3. *Cistus monspeliensis* extract for use according to claim 1 or 2, wherein the *Cistus monspeliensis* extract is obtained from the flowering aerial parts of the plant.

4. *Cistus monspeliensis* extract for use according to any one of claims 1 to 3, wherein the *Cistus monspeliensis* extract is obtained by supercritical CO₂ extraction, preferably with the addition of ethanol as co-solvent.

5. *Cistus monspeliensis* extract for use according to claim 1, for use in the prevention or treatment of skin pruritus.

6. Dermatological composition comprising as active principle an extract of *Cistus monspeliensis,* with at least one dermatologically acceptable excipient, for use in the prevention and/or treatment of skin disorders associated with cutaneous neurogenic inflammation, the skin disorders being pruritic inflammatory dermatoses.

7. A dermatological composition for use according to claim 6, wherein the pruritic inflammatory dermatosis is atopic dermatitis.

8. Dermatological composition for use according to claim 6 or 7, wherein the extract of *Cistus monspeliensis* is obtained from the flowering aerial parts of the plant.

9. Dermatological composition for use according to any one of claims 6 to 8, wherein the *Cistus monspeliensis* extract is obtained by extraction with supercritical CO2.

10. Dermatological composition for use according to any one of claims 6 to 9, wherein the *Cistus monspeliensis* extract is obtained by supercritical CO2 extraction with the addition of ethanol as co-solvent.

11. Dermatological composition for use according to any one of claims 6 to 10, wherein it comprises 0.01 to 5%, preferably 0.02 to 2%, of *Cistus monspeliensis* extract by weight of dry extract relative to the total weight of the composition.
